(19) Europäisches Patentamt
European Patent Office
Office européen des brevets

(11) Publication number: **0 203 621 B1**

(12) **EUROPEAN PATENT SPECIFICATION**

(45) Date of publication of patent specification: **07.03.90**

(51) Int. Cl.[5]: **C 07 H 17/08**, A 61 K 31/70, A 23 K 1/17

(21) Application number: **86108251.9**

(22) Date of filing: **08.09.83**

(60) Publication number of the earlier application in accordance with Art. 76 EPC: **0104028**

# (54) C-20-modified macrolide derivatives.

(30) Priority: **13.09.82 US 417248**

(43) Date of publication of application: **03.12.86 Bulletin 86/49**

(45) Publication of the grant of the patent: **07.03.90 Bulletin 90/10**

(84) Designated contracting states: **BE CH DE FR GB IT LI LU NL SE**

(56) References cited:
**EP—A— 0 087 921**
**GB—A— 2 058 765**
**CHEM. PHARM. BULL., vol. 30, no. 1, 1982, pages 97-110; H. MATSUBARA et al.: "Chemical transformation of tylosin, a 16-membered macrolide, and its structure-activity relationship"**

(73) Proprietor: **ELI LILLY AND COMPANY**
**Lilly Corporate Center**
**Indianapolis Indiana 46285 (US)**

(72) Inventor: **Kirst, Herbert Andrew**
**1819 Madison Village Apt. B-6**
**Indianapolis Indiana 46227 (US)**
Inventor: **Toth, John Eldon**
**121 West Wood Street Apt. 5**
**West Lafayette Indiana 47906 (US)**

(74) Representative: **Tapping, Kenneth George et al**
**Erl Wood Manor**
**Windlesham Surrey, GU20 6PH (GB)**

**EP 0 203 621 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

This invention provides a group of compounds which are C-20-modified derivatives of the macrolide antibiotics tysolin, desmycosin, macrocin, lactenocin, 2‴-O-demethylmacrocin (DOMM) and 2″-O-demethyl-lactenocin (DOML). The compounds are useful as antibiotics or as intermediates to antibiotics.

Certain C-20-modified tylosin derivatives are disclosed in Chem. Pharm. Bull. 30 (1) 1982 pp. 97-110, in GB-A-2058765 and in EP-A-0087921, the latter falling under EPC Art. 54 (3).

Other C-20 modified tylosin derivatives are claimed in our divisional application EP-A-0203621.

More specifically, the present invention provides macrolides of the formula (I)

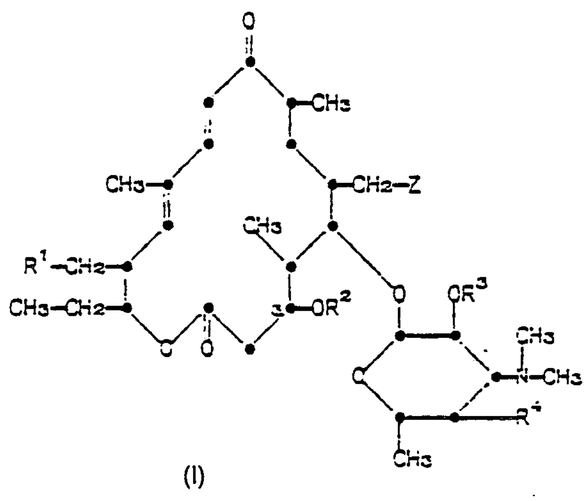

(I)

wherein

$R^1$ is 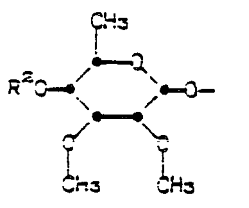 ; 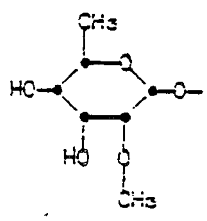 ; or 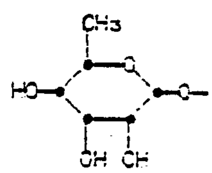

$R^2$ is hydrogen or a hydroxyl protecting group ;

$R^3$ is hydrogen, optionally substituted $C_1$-$C_5$-alkanoyl optionally substituted benzoyl, optionally substituted phenylacetyl or optionally substituted phenylpropionyl ;

$R^4$ is hydrogen, iodo, hydroxy, optionally substituted $C_1$-$C_5$-alkanoyloxy, optionally substituted benzoyloxy, optionally substituted phenylacetoxy or optionally substituted phenoxyacetoxy or

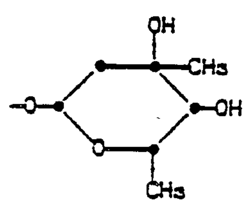

(mycarosyloxy)

Z is a group

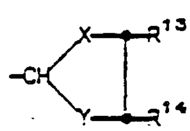

or

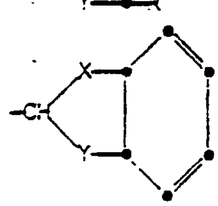

where X and Y independently represent O, S, NH, $NCH_3$, N-phenyl, or N-benzyl and $R^{13}$ and $R^{14}$ are independently hydrogen, methyl, phenyl, methoxycarbonyl, ethoxycarbonyl, or phenoxycarbonyl, provided that if $R^4$ is hydrogen or iodo, then

$R^1$ is 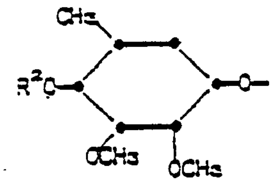

and provided that $R^2$ is hydrogen unless $R^4$ is hydrogen or iodo.

Although no stereochemical assignments are indicated in formula (I), it is to be understood that the stereochemistry is that of tylosin.

The term « $C_1$-$C_5$-alkanoyl » as used herein refers to an acyl moiety derived from a carboxylic acid containing from one to five carbon atoms. In such a moiety, the alkyl group can be straight, branched, or cyclic. When optionally substituted, the alkyl group can bear one to three halo substituents. Halo substituents are selected from the group consisting of Cl, Br and F. Acetyl, chloroacetyl, trichloroacetyl, trifluoroacetyl, propionyl, n-butyryl, isobutyryl, n-valeryl, and isovaleryl are examples of such groups. The term « $C_1$-$C_5$-alkanoyloxy » refers to the corresponding acyloxy moiety.

The terms « optionally substituted benzoyl, phenylacetyl, phenylpropionyl, phenoxyacetyl of phenylthioacetyl », « optionally substituted benzoyl, phenylacetyl or phenylpropionyl », « optionally substituted benzoyloxy, phenylacetoxy or phenoxyacetoxy », « optionally substituted phenyl, benzyl or phenethyl », « optionally substituted benzyl, phenethyl or phenoxyethyl » and « optionally substituted phenyl » mean that the phenyl portion of the moiety is optionally substituted by from one to five halo or methyl groups of by from one to two methoxyl, nitro or hydroxyl groups.

The term « hydroxyl-protecting group » refers to a substituent which is not removed under the reaction conditions but which can be readily removed after the reaction has been completed to liberate the original hydroxyl group. Hydroxyl-protecting groups are well known in the art (see, for example, T. W. Greene, « Protective Groups in Organic Synthesis », Wiley-Interscience, 1981, pp. 10-86). One especially suitable hydroxyl-protecting group is the tetrahydropyranyl group.

Preparing Macrolides of Formula (I)

Macrolides of formula (I) are prepared from corresponding macrolides of formula (II)

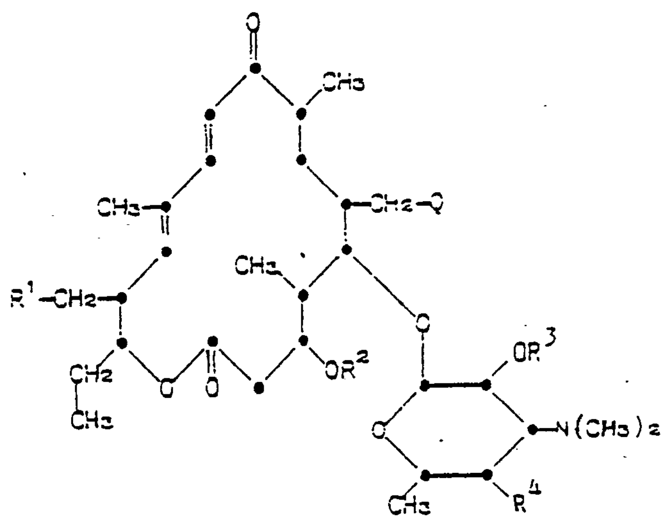

(II)

where $R^1$, $R^2$, $R^3$, and $R^4$ are as previously defined in formula (I) and Q is CHO.

Aldehyde starting materials, i.e., macrolides of formula (II) wherein Q is —CHO include the following known macrolides of formula (II) and esters thereof :

(See Table page 4)

| | $R^2$ | $R^3$ | $R^4$ | $R^1$ |
|---|---|---|---|---|
| tylosin | H | H | mycarosyloxy | mycinosyloxy |
| desmycosin | H | H | OH | mycinosyloxy |
| 4'-deoxydesmycosin | H | H | H | mycinosyloxy |
| macrocin | H | H | mycarosyloxy | 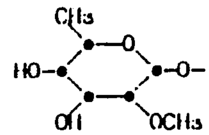 |
| lactenocin | H | H | OH | 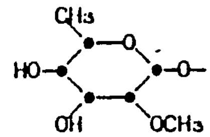 |
| 2'''-O-demethylmacrocin (DOMM) | H | H | mycarosyloxy | 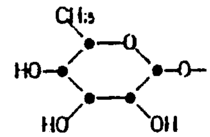 |
| 2'''-O-demethylactenocin (DOML) | H | H | OH | 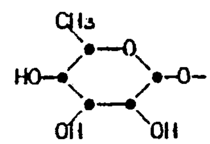 |

Tylosin and its prepation by fermentation of Streptomyces fradiae NRRL 2702 or 2703 are described in U.S. Patent No. 3,178,341. Desmycosin and preparation of desmycosin by mild acid hydrolysis of tylosin are also described in U.S. Patent No. 3,178,341.

4'-Deoxydesmycosin and a method of preparing it from desmycosin are described in A. Tanaka et al., J. Antibiotics 34, 1381-84 (1981).

Macrocin and its preparation by fermentation of Streptomyces fradiae NRRL 2702 or 2703 are described in U.S. Patent No. 3,326,759. Lactenocin and preparation of lactenocin by mild acid hydrolysis of macrocin are also described in U.S. Patent No. 3,326,759.

DOMM and its preparation by fermentation of Streptomyces fradiae ATCC 31669 are described in EPO published specification 45157. DOML and preparation of DOML by mild acid hydrolysis of DOMM are also described in EPO published specification 45157.

Aldehyde starting materials of formula (II) which are 2'- or 4'-monoesters, or 2',4'-diesters of the above described known macrolides, i. e., macrolides of formula (II) wherein $R^3$ is other than hydrogen or $R^4$ is other than hydroxyl, or both, are prepared by known acylation procedures. Typical acylating agents include activated carboxylic acid derivatives such as anhydrides, acid halides (usually in combination with a base or other acid scavenger) and active esters. Suitable organic solvents for this reaction include pyridine and triethylamine. Acylation can also be achieved using a mixture of an organic acid and a dehydrating agent such as N,N'-dicyclohexylcarbodiimide. Once formed, the acyl derivative can be separated and purified by known techniques.

Aldehyde starting materials of Formula (II) which are 2'-monoester derivatives can be prepared by selective esterification of compounds of formula (II) wherein $R^3$ is hydrogen and $R^4$ is hydroxyl using the technique described in U.S. Patents 4,321,361 and 4,321,362. 2'-Hydroxyl groups are more facile to esterification than 4'-hydroxyl groups. Accordingly, the 2'-monoester derivative can be selectively prepared by, for example, treating the macrolide starting material with a stoichiometric quantity (or a slight excess) of an acylating agent, such as an acyl anhydride, at about room temperature for from about 1 to about 24 hours until esterification is substantially complete. The 2'-monoester can be isolated from the reaction mixture by standard procedures such as extraction, chromotography and crystallization.

Aldehyde starting materials which are 2',4'-diester derivatives are prepared from macrolides of formula (II) wherein $R^3$ is hydrogen and $R^4$ is hydroxyl using the procedure described in published European Patent specification 82,003. Thus, symmetrical 2',4'-diester derivatives are prepared by treating the known macrolide of formula (II) wherein $R^3$ is hydrogen and $R^4$ is hydroxyl with a stoichiometric quantity (or a slight excess) of an acylating agent, such as an acyl anhydride, in a neutral solvent such as acetone, at about room temperature for from 1 to about 24 hours until esterification of the 2' and 4' hydroxyl groups is complete. Unsymmetrical 2',4'-diester derivatives, i. e., compounds of formula (II) wherein $OR^3$ and $R^4$ are different, can be prepared by acylation of appropriate 2'-monoesters.

Macrolides of formula (I) wherein Z is a group

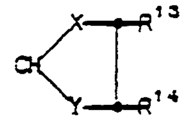

(VII)

or

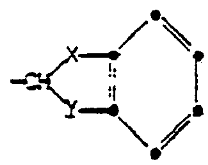

(VIII)

wherein X, Y, $R^{13}$, and $R^{14}$ are as previously defined in formula (I) are prepared by reacting a macrolide of formula II wherein Q is —CHO with a compound of formula

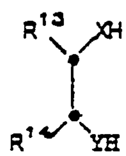

(XI) or

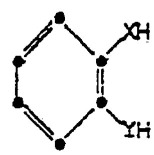

(XII)

in a nonreactive organic solvent, such as benzene, tetrahydrofuran, acetone, methylene chloride, or acetonitrile. In some case, addition of an acidic catalyst, for example p-toluenesulfonic acid, or continuous removal of water may be necessary to drive the reaction to completion. Molecular sieves may advantageously be used to remove water. Suitable reaction conditions for this reaction are known in the art and have been summarized in T.W. Greene, « Protective Groups in Organic Synthesis », Wiley-Interscience, 1981, pp 116-141.

When a product of formula (I) is one in which $R^4$ is mycarosyloxy, the corresponding macrolide of formula (I) wherein $R^4$ is hydroxyl can be prepared by acid hydrolysis of the initial product. More specifically, the mycarose sugar can be hydrolytically cleaved at a pH of less than 4, preferably in the range from 0.5 to 2.0, at a temperature in the range of from 0 to 60 °C, conveniently at about room temperature. The hydrolysis can be effected using a strong aqueous mineral acid such as hydrochloric or sulfuric acid or a strong organic acid such as p-toluenesulfonic acid.

As previously mentioned, a method of preparing 4′-deoxydesmycosin is described in J. of Antibiotics 34, 1381-84 (1981). The process involves (1) treatment of desmycosin with acidic ethanol in accordance with a procedure described in Antibiot. & Chemoth. 11, 320-27 (1961), to obtain the corresponding diethylacetal ; (2) acylation of the diethylacetal with acetic anhydride in acetonitrile in the absence of external base, in accordance with a procedure described in J. Org. Chem. 44, 2050-52 (1979), to obtain the 2′,4′-di-O-acetyl derivative ; (3) reacting the 2′,4′-di-O-acetyl derivative with 2,3-dihydrofuran in dichloromethane in the presence of pyridinium p-toluene-sulfonate in the manner described in J. Org. Chem. 42, 3772-74 (1974) to obtain the 3,4″-bis (O-tetrahydrofuranyl) derivative ; (4) removal of the 2′ and 4′-O-acetyl groups by dissolving the product of step (3) in methanol (50 °C, overnight) ; (5) reacting the product of step (4) with 1.5 mole equivalent of benzenesulfonyl chloride in pyridine at —40 °C for 4 hours, to provide the 4′-O-benzenesulfonyl derivative ; (6) immediately reacting the 4′-O-benzenesulfonyl derivative with 1.5 equivalent of sodium iodide in methyl ethyl ketone at 180 °C. for 15 minutes to obtain 4′ iodo derivative ; (7) reductively deiodinating the 4′-iodo derivative using tri(n-butyl) stannane in benzene in the presence of 2,2′-azobis-isobutyronitrile at 80 °C for 2 hours ; and (8) deblocking the diethylacetal and tetrahydrofuranyl groups by hydrolysis of the product of step (7) in 1M aqueous hydrochloric acid-acetonitrile (2.5 : 1 v/v) for 30 minutes at 25 °C to obtain 4′-deoxydesmycosin.

The 4′-deoxydesmycosin thus prepared may then be modified at the C-20, and optionally at the 2′ position as described above.

Alternatively, a C-20 modified derivative of 4′-deoxydesmycosin may be prepared by deoxygenating a C-20 modified derivative of desmycosin, for example by treating the C-20 modified derivative in accordance with steps 2 through 6 or 2 through 8 of the process of J. Antibiotics 34, 1381-84 (1981) as described above.

The procedures used to prepare 2′ and 4′ esters of the starting macrolides of formula (II) were described above. Macrolides of formula (I) can be acylated using identical procedures to obtain 2′- and 4′-monoesters and 2′,4′-diesters of formula (I).

The C-20-modified derivatives of this invention form salts, particularly acid addition salts. These acid addition salts are also useful as antibiotics and are a part of this invention. In another aspect, such salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicyclic, methanesulfonic, benzenesulfonic, sorbic, picric, benzoic, cinnamic, and like acids.

Accordingly, the present invention provides a process for preparing a macrolide of formula (I) or a pharmaceutically-acceptable salt thereof, which comprises reacting a starting macrolide of formula (II)

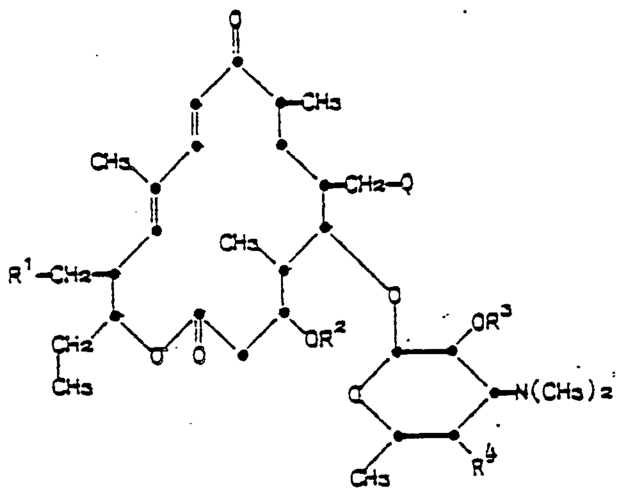

(II)

wherein $R^1$, $R^2$, $R^3$, and $R^4$ are as defined in formula (I), and where Q is —CHO with a compound of formula

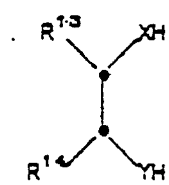 (XI) or 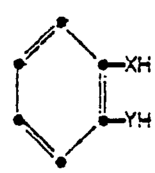 (XII)

where $R^{13}$, $R^{14}$, X and Y are as defined in formula (I) to provide a macrolide of formula (I) wherein Z is

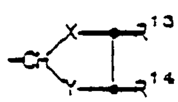

(VII)

or

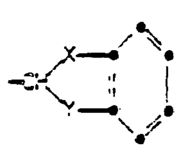

(VIII)

or hydrolyzing a macrolide of formula (I) wherein $R^4$ is mycarosyloxy in acid solution at a pH below 4 to provide a corresponding macrolide of formula (I) wherein $R^4$ is hydroxy; or reacting a macrolide of formula (I) wherein $R^4$ is a sulfonate with a source of iodide ion to provide a macrolide of formula (I) wherein $R^4$ is iodo; or reductively deiodinating a macrolide of formula I wherein $R^4$ is iodo to provide a macrolide of formula (I) wherein $R^4$ is hydrogen; and optionally esterifying or salifying the macrolide provided by any of the foregoing steps.

Illustrative C-20-modified derivatives of this invention are listed in Table VIII.

Table VIII

Illustrative C-20-Modified Derivatives of Desmycosin[a]

| Compound No. | Z |
|---|---|
| 74 | 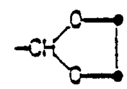 |
| 75 | 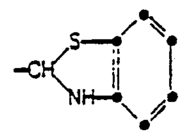 |
| 76 | 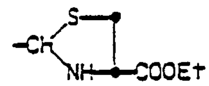 |
| 77 | 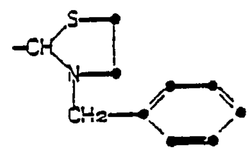 |
| 78 | 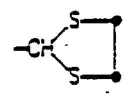 |
| 79 | 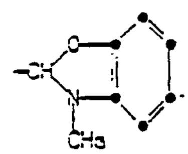 |
| 80 | 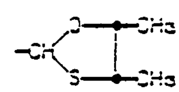 |

a $R^1$ = 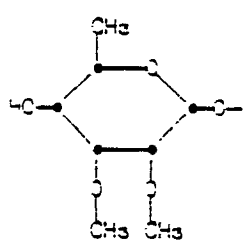 ; $R^3$ = H; $R^4$ = OH

The derivatives of this invention inhibit the growth of pathogenic bacteria, especially gram-positive bacteria, Mycoplasma species and Pasteurella species. The derivatives are particularly useful against the Pasteurella species P. multocida and P. hemolytica and against Mycoplasma hyopneumoniae, the causative agent of mycoplasmal pneumonia in swine, and Mycoplasma gallisepticum.

In addition, many of the C-20-modified compounds of this invention exhibit higher blood levels than those of the parent compounds.

The minimal inhibitory concentrations (MIC's) at which illustrative compounds inhibit certain bacteria are given in Tables IX and X. The MIC's in Table IX were determined by standard agar-dilution assays. The MIC's in Table X were obtained using conventional broth-dilution microtiter tests.

Table IX

Antibiotic Activity of C-20 Modified Derivatives[a]

| Test Organism | Test Compound[b] 74 | 75 | 76 | 77 |
|---|---|---|---|---|
| *Staphylococcus aureus* X1.1 | 0.5 | 0.25 | 0.5 | 0.25 |
| " " V41[c] | 1 | 0.25 | 0.5 | 0.25 |
| " " X400[d] | 2 | 0.5 | 2 | 0.25 |
| " " S13E | 0.5 | 0.25 | 0.5 | 0.25 |
| *Staphylococcus epidermidis* EPI1 | 0.5 | 0.25 | 0.5 | 0.25 |
| " " EPI2 | 0.25 | 0.12 | 0.25 | 0.12 |
| *Streptococcus pyogenes* C203 | NT | 0.5 | 0.5 | 0.25 |
| " *pneumoniae* Park I | 0.25 | 0.25 | 0.25 | 1 |
| " Group D X66 | 8 | 2 | 4 | 4 |
| " " 9960 | 8 | 2 | 4 | 4 |
| *Haemophilus influenzae* Holt[e] | 32 | 16 | 32 | 16 |
| " " R252[f] | 32 | 16 | 32 | 16 |

[a]MIC in mcg/ml
[b]Compound numbers from Tables I-VIII
[c]Penicillin-resistant strain
[d]Methicillin-resistant strain
[e]Ampicillin-sensitive strain
[f]Ampicillin-resistant strain
[g]Not tested
[h]Not active at 128 mcg/ml, the highest level tested

Table X

Antibiotic Activity of C-20 Modified Derivatives[a]

| Test Organism | Test Compound[b] 74 | 75 | 76 | 77 |
|---|---|---|---|---|
| *Staphylococcus aureus* | 3.12 | 0.78 | 0.78 | 0.39 |
| *Streptococcus bovis* 80 | 6.25 | 3.12 | 3.12 | 3.12 |
| *Pasteurella multocida* 17E[c] | 25 | 25 | 25 | 12.5 |
| " " 60A[d] | 50 | 25 | 25 | 12.5 |
| *Pasteurella hemolytica* 22C | 50 | 25 | 25 | 12.5 |
| *Mycoplasma gallisepticum* | 3.12 | 1.56 | 1.56 | 0.09 |
| " *synoviae* | 6.25 | 1.56 | 3.12 | 0.19 |
| " *hyorhinis* | 50 | 50 | 50 | 6.25 |
| " *hyopneumoniae* | 1.56 | 0.19 | 0.19 | 0.19 |

[a]MIC in mcg/l
[b]Compound numbers from Tables I-VIII
[c]Bovine isolate
[d]Avian isolate
[e]Not active at 50 mcg/ml, the highest level tested
[f]Not tested

The C-20 modified derivatives of this invention have shown in vivo antimicrobial activity against experimentally-induced infections in laboratory animals. When two doses of test compound were administered to mice experimentally infected with S. pyogenes C203, the activity observed was measured as an $ED_{50}$ value [effective dose in mg/kg to protect 50 % of the test animals : see Warren Wick, et al., J. Bacteriol. 81, 233-235 (1961)]. $ED_{50}$ values observed for illustrative compounds are given in Table XI.

Table XI

$ED_{50}$ Values of C-20 Modified Derivatives[a]

| Test Compound[b] | Streptococcus pyogenes C203 | |
|---|---|---|
| | Subcutaneous | Oral |
| 74 | 3.3 | >25 |
| 75 | 1.7 | 30 |
| 76 | 4.8 | 17 |
| 77 | >10 | 19 |

[a]mg/kg × 2 ; doses given 1 and 4 hours post-infection
[b]Compound numbers from Tables I-VIII

Many of the C-20 modified derivatives of this invention have also shown in vivo antibacterial activity against infections induced by gram-negative bacteria. Tables XII and XIII summarize the results of tests in which illustrative compounds were evaluated against Pasteurella infection in one-day-old chicks. The compounds were administered parenterally or orally after challenge of the chicks with Pasteurella multocida (0.1 ml of a 10-4 dilution of a twenty-hour tryptose broth culture of an avian P. multocida given subcutaneously). In these tests, unless indicated otherwise, all non-medicated infected chicks died within 24 hours of Pasteurella challenge. In the tests summarized in Table XII the compounds were administered by subcutaneous injection at a dosage of 30 mg/kg, 1 and 4 hours post-challenge of the chicks with P. multocida. In the tests summarized in Table XIII the compounds were administered by gavage at 1 and 5 hours post-challenge of the chicks with P. multocida.

Table XII

Activity of C-20-Modified Derivatives Administered Subcutaneously to Pasteurella multocida-Infected Chicks[a]

| Test Compound[b] | Number of Deaths/Number Treated |
|---|---|
| 75 | 4/10 |
| 76 | 3/10 |
| 77 | 8/10[d] |

[a]Administered subcutaneously ; 30 mg/kg × 2
[b]Compound numbers from Tables I-VIII
[d]8/10 and 9/10 of two groups of infected non-medicated chicks died in this test

(See table XIII page 10)

Table XIII

Activity of C-20 Modified Derivatives Administered Orally to Pasteurella multocida-Infected Chicks[a]

| Test Compound[b] | Dose (g/gal)[c] | Number of Deaths/ Number tested |
|---|---|---|
| 75 | 2.0 | 3/9 |
| 75 | 1.0 | 6/9 |
| 75 | 2.0 | 4/10 |
| 76 | 0.5 | 3/10 |
| 76 | 1.0 | 4/10[d] |
| 76 | 2.0 | 4/10[e] |
| 77 | 2.0 | 4/10[e] |

[a]Administered by gavage
[b]Compound number from Tables I-VIII
[c]Dose administered in 0.1-ml of solution equivalent to the presumed amount of compound which would be consumed by chicks drinking medicated water at the concentration indicated over a 24-hour period
[d]13/20 Infected non-medicated chicks died in this test
[e]11/20 Infected non-medicated chicks died in this test

The compounds of this invention have also exhibited in vivo activity against experimental infections caused by Mycoplasma gallisepticum. In these tests infections were induced in chicks by injecting 0.2 ml of a broth culture of M. gallisepticum into the abdominal air sac of two- to three-day-old chicks. The compounds were administered by gavage five times at a dose equivalent to 0.5 g/gal (on the day of challenge once prior to and once following the challenge, two times on the next day and once on the third day). Twenty-one days post-infection, the chicks were weighed, a blood sample was taken, and the chicks were sacrificed. The presence or absence of air-sac lesions was recorded. The results of these tests are summarized in Table XIV.

Table XIV

Antimycoplasmal Activity of C-20-Modified Derivatives in Chicks

| Test Compound[a] | Mortality | Number with Air-Sac Lesions/ Number Treated | Number with Antibodies[b]/ Number Tested |
|---|---|---|---|
| 75 | 1/10 | 3/10 | 9/9 |
| Infected Control | 2/10 | 10/10 | 8/8 |
| Normal Control | 0/0 | 0/0 | 0/0 |

[a]Compound numbers from Tables I-VIII
[b]Antibodies to M. gallisepticum

This invention is useful in methods of controlling infections caused by bacterial and mycoplasmal species. In carrying out these methods, an effective amount of a compound of formula I is administered parenterally or orally to an infected or susceptible warm-blooded animal. The compounds can also be administered by insufflation, i. e. by blowing the compound, in the form of a medicated dust, into an enclosed space or room wherein the animals or poultry are held. The animals or poultry breathe the medicated dust present in the air; the medicated dust is also taken into the body through the eyes (a process called intraocular injection).

The dose which is effective to control the infection will vary with the severity of the infection and the age, weight, and condition of the animal. The total dose required for protection parenterally will generally, however, be in the range of from about 1 to about 100 mg/kg and preferably will be in the range of from about 1 to about 50 mg/kg. The dose required for oral administration will generally be in the range of from

1 to about 300 mg/kg and preferably will be in the range of from about 1 to about 100 mg/kg. Suitable dosage regimens can be constructed.

Often the most practical way to administer the compoounds is by formulation into the feed supply or drinking water. A variety of feeds, including the common dry feeds, liquid feeds, and pelleted feeds, may be used.

In another aspect, this invention relates to compositions useful for the control of infections caused by bacteria and Mycoplasma species. These compositions comprise a compound of formula I together with a suitable vehicle. Compositions may be formulated for parenteral or oral administration by methods recognized in the pharmaceutical art.

The methods of formulating drugs into animal feeds are well-known. A preferred method is to make a concentrated-drug premix which in turn is used to prepare medicated feeds. Typical premixes may contain from about 1 to about 200 grams of drug per pound of premix. Premixes may be either liquid or solid preparations.

The final formulation of feeds for animals or poultry will depend upon the amount of drug to be administered. The common methods of formulating, mixing, and pelleting feeds may be used to prepare feeds containing a compound of formula I.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50 % of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, and surface tension of the liquid suspending medium can assist in making injectable suspensions in individual cases. For example, silicone antifoams, sorbitol, and sugars can be useful suspending agents.

In order to illustrate more fully the operation of this invention, the following examples are provided. In these examples the abbreviations « 20-DH » and « 20-DH-DO » are used for the terms « 20-dihydro » and « 20-dihydro-20-deoxy », respectively.

## Preparation 1

20-Dihydrotylosin (Relomycin)

A solution of tylosin base (30.0 g, 32.8 mmole) in 2-propanol (300 ml) and water (200 ml) was treated with sodium borohydride (315 mg, 8.2 mmole), portionwise, over five minutes. Thirty minutes after the addition was completed, the pH of the reaction solution was adjusted to 7.0 by the addition of a 1N sulfuric acid solution. The neutralized solution was evaporated under vacuum to remove the 2-propanol; the aqueous solution remaining was treated with a saturated sodium bicarbonate solution (500 ml). The mixture was extracted with dichloromethane (3 × 300 ml), and the combined extract was washed with a saturated sodium chloride solution (1 × 200 ml) and dried over sodium sulfate. Filtration followed by evaporation gave a glass which was broken up in n-hexane, collected on a filter and air dried to yield 28.5 g (95 %) of 20-dihydrotylosin.

## Preparation 2

20-Dihydrodesmycosin

Desmycosin (10 g, 13 mmoles), dissolved in isopropanol : water (1 : 1, 175 ml), was stirred at room temperature while $NaBH_4$ (125 mg, 3.3 mmoles) was added. After 1/2 hour the pH of the reaction mixture was adjusted to 7.0 with 1N $H_2SO_4$. The alcohol was removed under reduced pressure. Saturated $NaHCO_3$ solution was added to the aqueous solution, and the product was extracted into $CH_2Cl_2$. The organic layer was dried ($Na_2SO_4$), and solvent was removed under reduced pressure to give 9.65 g of 20-dihydrodesmycosin (12.5 mmoles, 96 % yield) as a white foam.

Preparation 3

20-DH-DO-20-iododesmycosin (Method 1)

20-Dihydrodesmycosin (2.0 g, 2.6 mmoles) and tetra n-butylammonium iodide (1.5 g, 3.9 mmoles) were dissolved in $CH_2Cl_2$ (30 ml) with s-collidine (0.6 ml, 4.5 mmoles) added. This solution was cooled to —78 °C under a nitrogen atmosphere and treated with trifluoromethanesulfonic anhydride (0.6 ml, 3.9 mmoles) dropwise by syringe. The reaction was stirred for 5 minutes at —78 °C and then allowed to come to room temperature (about 30 minutes). Saturated $NaHCO_3$ solution was added, and the product was extracted with $CH_2Cl_2$. The organic layer was dried ($Na_2SO_4$) and evaporated to give a red oil which was purified by silica-gel flash chromatography, eluting initially with $CH_2Cl_2$ (400 ml) and then stepwise with $CH_2Cl_2 : CH_3OH$ solutions as follows : 98 : 2 (250 ml) ; 96 : 4 (500 ml) 95 : 5 (250 ml) ; 94 : 6 (750 ml) and 92 : 8 (250 ml). Fractions containing the desired product were identified by TLC, combined and evaporated to dryness to give 20-DH-DO-20-iododesmycosin (595 mg, 0.67 mmoles, 26 % yield) as a white foam.

Preparation 4

20-DH-DO-20-iododesmycosin (Method 2)

20-Dihydrodesmycosin (5.0 g, 6.5 mmoles) and triphenylphosphine (2.54 g, 9.70 mmoles) were dissolved in dimethylformamide (DMF) (10 ml). This mixture was stirred at room temperature under $N_2$ while iodine (2.46 g, 9.70 mmoles) in DMF (5 ml) was added dropwise. The reaction mixture was stirred for two hours and then poured into cold saturated $NaHCO_3$ solution. The product was extracted with $CHCl_3$ (two portions) and the combined $CHCl_3$ extracts were shaken with 0.1M sodium thiosulfate to remove unreacted iodine. The organic layer was dried ($Na_2SO_4$) and evaporated under reduced pressure to give a light yellow oil which was purified by silica-gel flash chromatography. The column was eluted initially with $CH_2Cl_2$ (500 ml) and then with 250 ml portions of $CH_2Cl_2 : CH_3OH$ mixtures as follows : 98 : 2 ; 96 : 4 ; 95 : 5 ; 94 : 6 ; 92 : 8 ; 88 : 12 ; and 86 : 14. Fractions containing the desired product were identified as in Preparation 2 and combined to give 1.78 g (2.0 mmoles, 31 % yield) of 20-DH-DO-20-iododesmycosin as a white foam.

Preparation 5

20-DH-20-O-(p-Toluenesulfonyl) tylosin

A solution of 20-dihydrotylosin (10.0 g, 10.9 mmole) and 4-(N,N-dimethylamino) pyridine (24 mg, 0.2 mmole) in dichloromethane (100 ml) and pyridine (10 ml) was treated with p-toluenesulfonyl chloride (2.08 g, 10.9 mmole). The resulting solution was stirred at room temperature with the exclusion of moisture. Additional p-toluenesulfonyl chloride was added after three hours (1.0 g, 5.2 mmole) and after twenty-two hours (240 mg, 1.3 mmole). After twenty-seven hours, methanol (0.8 ml) was added, and the solution was evaporated to give a glass. The glass was dissolved in dichloromethane, washed with saturated sodium bicarbonate solution and dried over sodium sulfate. The solution was filtered and then evaporated to give a glass that was purified by silica-gel flash chromatography. Elution with a gradient of 1 L of dichloromethane to 1 L of methanol/dichloromethane (3.5 : 96.5) and then with 2 L of methanol/dichloromethane (3.5 : 96.5) gave 5.37 g (46 %) of pure 20-DH-DO-20-O-(p-toluenesulfonyl) tylosin and 2.3 g (20 %) of slightly impure 20-DH-20-O-(p-toluenesulfonyl) tylosin.

Example 1

Deformyl-19-(1,3-Dioxolan-2-yl) desmycosin

A solution of desmycosin (3.0 g, 3.89 mmole) in acetonitrile (20 ml) and ethylene glycol (15 ml) was treated with powdered 4A molecular sieves and p-toluenesulfonic acid monohydrate (1.11 g, 5.84 mmole) and stirred at room temperature with the exclusion of moisture for one hour. The reaction mixture was neutralized by the addition of solid sodium bicarbonate (600 mg) and filtered. The filtrate was poured into a saturated sodium bicarbonate solution. The resulting solution was extracted three times with dichloromethane. The extracts were combined, washed with saturated sodium chloride solution and then with water, dried over sodium sulfate, filtered and evaporated to give a glass. The glass was purified by silica-gel flash chromatography, eluting with a gradient of 1 L of dichloromethane to 1 L of methanol/dichloromethane (1 : 9) and then with 500 ml of methanol/dichloromethane (1 : 9) to give 1.46 g (46 %) of deformyl-19-(1,3-dioxolan-2-yl) desmycosin.

Example 2

Deformyl-19-(Benzothiazolidin-2-yl) desmycosin

A solution of desmycosin (2.0 g, 2.6 mmole) in dichloromethane (40 ml) was treated with 2-aminothiophenol (400 mg, 2.9 mmole) and stirred at room temperature with the exclusion of moisture for twenty minutes. The reaction was washed with saturated sodium bicarbonate solution, dried over sodium sulfate, filtered and evaporated to give a glass. The glass was purified by silica-gel flash chromatography, eluting with a gradient of 1 L of dichloromethane to 1 L of methanol/dichloromethane (1 : 9) to give 1.8 g (79 % yield) of deformyl-19-(benzothiazolidin-2-yl) desmycosin.

Example 3

Injectable Formulations

A) A formula I base is added to propylene glycol. Water and benzyl alcohol are added so that the solution contains 50 % (by volume) propylene glycol, 4 % (by volume) benzyl alcohol, and 200 mg/ml of a formula I base.

B) A solution is prepared as described in Section A except that the solution contains 50 mg/ml of a formula I base.

C) A solution is prepared as described in Section A except that the solution contains 350 mg/ml of a formula I base.

D) A solution is prepared as described in Section A except that the solution contains 500 mg/ml of a formula I tartrate.

E) A suspension is prepared by adding a finely ground formula I compound to carboxymethyl cellulose with thorough mixing so that the suspension contains 200 mg of the formula I base per ml of suspension.

Example 4

Chick Ration for Control of Mycoplasma

A balanced, high-energy ration adapted to feed chicks for rapid weight gain is prepared by the following recipe :

| Ingredient | % | lbs | kg |
|---|---|---|---|
| Ground yellow corn | 50 | 1,000 | (454) |
| Soybean meal, solvent-extracted dehulled, finely ground, 50 percent protein | 31.09 | 621.8 | (282) |
| Animal fat (beef tallow) | 6.5 | 130 | (59) |
| Dried fish meal, with solubles (60% protein) | 5.0 | 100 | (454) |
| Distillers' solubles from corn | 4.0 | 80 | (36.3) |
| Dicalcium phosphate, feed grade | 1.8 | 36 | (16.3) |
| Calcium carbonate | 0.8 | 16 | (7.3) |
| Vitamin premix (representing vitamins A, D, E, K, and $B_{12}$, choline, niacin, pantothenic acid, riboflavin, biotin, with glucose bulking agent) | 0.5 | 10 | (4.5) |

(Continued)

| Ingredient | % | lbs | kg |
|---|---|---|---|
| Trace mineral premix (representing $MnSO_4$, ZnO, KI, $FeSO_4$, $CaCO_3$) | 0.2 | 4 | (1.8) |
| 2-Amino-4-hydroxybutyric acid (hydroxy analog of methionine) | 0.1 | 2 | (0.9) |
| Formula I compound | 0.01 | 0.2 | (0.09) |

These substances are mixed in accordance with standard feed-mixing techniques. Chicks fed such a ration, with water ad libitum, are protected against exposure to Mycoplasma infections.

## Claims

1. A macrolide of the formula (I)

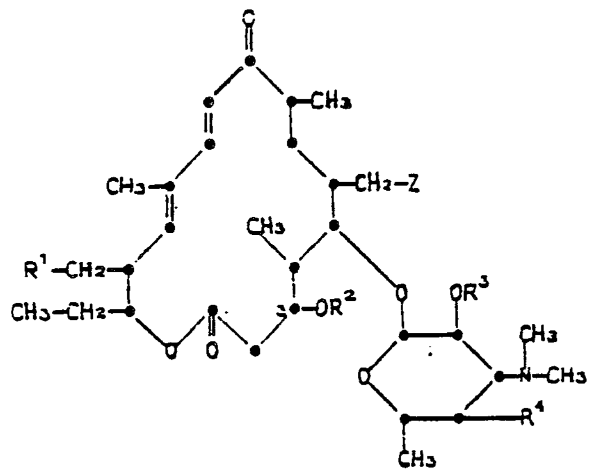

(I)

wherein

$R^1$ is 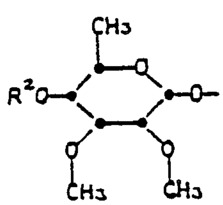 ; 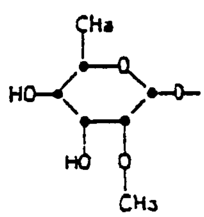 ; or 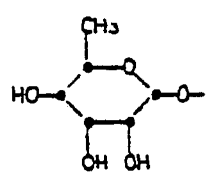

$R^2$ is hydrogen or a hydroxyl protecting group ;

$R^3$ is hydrogen, optionally substituted $C_1$-$C_5$-alkanoyl optionally substituted benzoyl, optionally substituted phenylacetyl or optionally substituted phenylpropionyl ;

$R^4$ is hydrogen, iodo, hydroxy, optionally substituted $C_1$-$C_5$-alkanoyloxy, optionally substituted benzoyloxy, optionally substituted phenylacetoxy or optionally substituted phenoxyacetoxy or

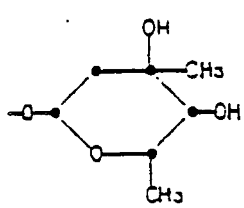

(mycarosyloxy)

Z is a group

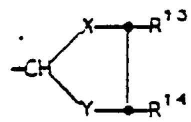

or

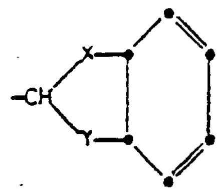

where X and Y independently represent O, S, NH, $NCH_3$, N-phenyl, or N-benzyl and $R^{13}$ and $R^{14}$ are independently hydrogen, methyl, phenyl, methoxycarbonyl, ethoxycarbonyl, or phenoxycarbonyl, provided that if $R^4$ is hydrogen or iodo, then R' is

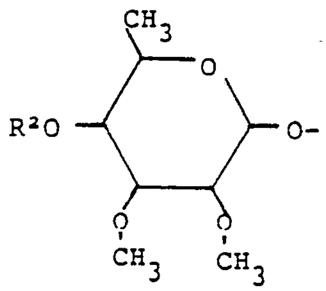

and provided that $R^2$ is hydrogen unless $R^4$ is hydrogen or iodo.

2. A process for preparing a macrolide of formula (I) or a pharmaceutically-acceptable salt thereof, which comprises reacting a starting macrolide of formula (II)

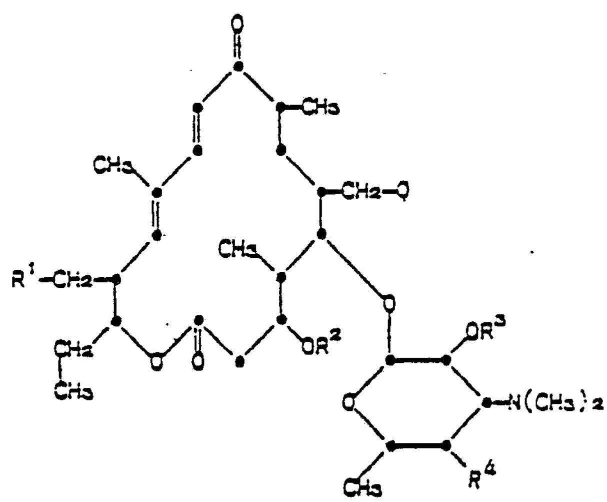

(II)

wherein $R^1$, $R^2$, $R^3$, and $R^4$ are as defined in formula (I), and where Q is —CHO with a compound of formula

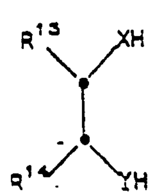

(XI) or

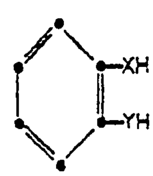

(XII)

where $R^{13}$, $R^{14}$, X and Y are as defined in formula (I) to provide a macrolide of formula (I) wherein Z is

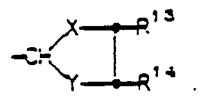

(VII)

or

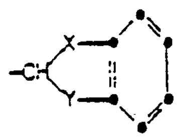

(VIII)

or hydrolyzing a macrolide of formula (I) wherein $R^4$ is mycarosyloxy in acid solution at a pH below 4 to provide a corresponding macrolide of formula (I) wherein $R^4$ is hydroxy ;
or reacting a macrolide of formula (I) wherein $R^4$ is a sulfonate with a source of iodide ion to provide a macrolide of formula (I) wherein $R^4$ is iodo ;
or reductively deiodinating a macrolide of formula I wherein $R^4$ is iodo to provide a macrolide of formula (I) wherein $R^4$ is hydrogen ; and
optionally esterifying or salifying the macrolide provided by any of the foregoing steps.

3. A macrolide of formula (I) or a pharmaceutically acceptable salt thereof as claimed in claim 1 for use as an antibiotic in the chemotherapy of warm-blooded animals.

4. A feed premix comprising as an active ingredient a macrolide of formula (I), or a pharmaceutically acceptable salt thereof, as claimed in claim 1.

5. A veterinary formulation which comprises as an active ingredient a macrolide of formula (I), or a pharmaceutically-acceptable salt thereof, as claimed in claim 1 associated with one or more physiologically-acceptable carriers or vehicles therefor.

**Patentansprüche**

1. Makrolid der Formel (I)

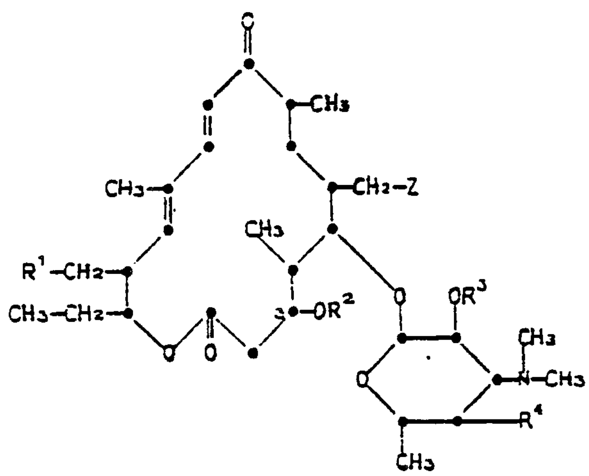

(I)

worin

$R^1$ folgende Gruppen bedeutet

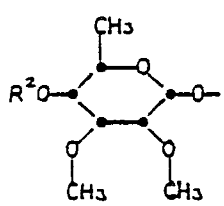 ; 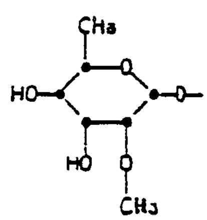 ; oder 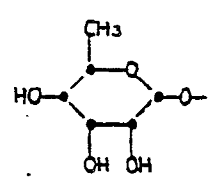 .

$R^2$ Wasserstoff oder eine Hydroxylschutzgruppe ist,

$R^3$ Wasserstoff, gegebenenfalls substituiertes $C_1$-$C_5$-Alkanoyl, gegebenenfalls substituiertes Ben-

zoyl, gegebenenfalls substituiertes Phenylacetyl oder gegebenenfalls substituiertes Phenylpropionyl bedeutet,

$R^4$ Wasserstoff, Iod, Hydroxy, gegebenenfalls substituiertes $C_1$-$C_5$-Alkanoyloxy, gegebenenfalls substituiertes Benzoyloxy, gegebenenfalls substituiertes Phenylacetoxy oder gegebenenfalls substituiertes Phenoxyacetoxy oder ein Rest der Formel

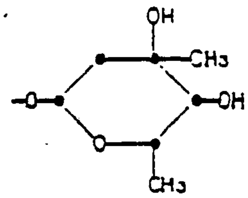

(Mycarosyloxy)

ist,

Z für eine der folgenden Gruppen

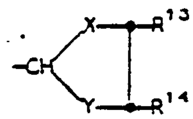

oder

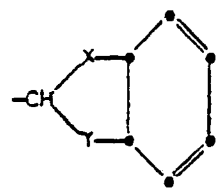

steht, worin X und Y unabhängig O, S, NH, $NCH_3$, N-Phenyl oder n-Benzyl sind und $R^{13}$ sowie $R^{14}$ unabhängig Wasserstoff, Methyl, Phenyl, Methoxycarbonyl, Ethoxycarbonyl oder Phenoxycarbonyl bedeuten, mit der Maßgabe, daß, falls $R^4$ Wasserstoff oder Iod ist, dann $R^1$ für eine Gruppe der Formel

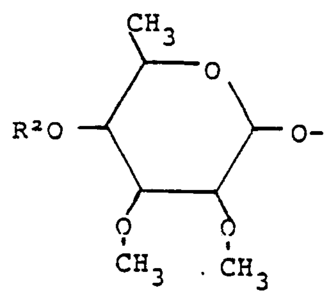

steht, und mit der weiteren Maßgabe, daß $R^2$ Wasserstoff ist, sofern $R^4$ nicht Wasserstoff oder Iod bedeutet.

2. Verfahren zur Herstellung eines Makrolids der Formel (I) oder eines pharmazeutisch annehmbaren Salzes hiervon, dadurch gekennzeichnet, daß ein Ausgangsmakrolid der Formel (II)

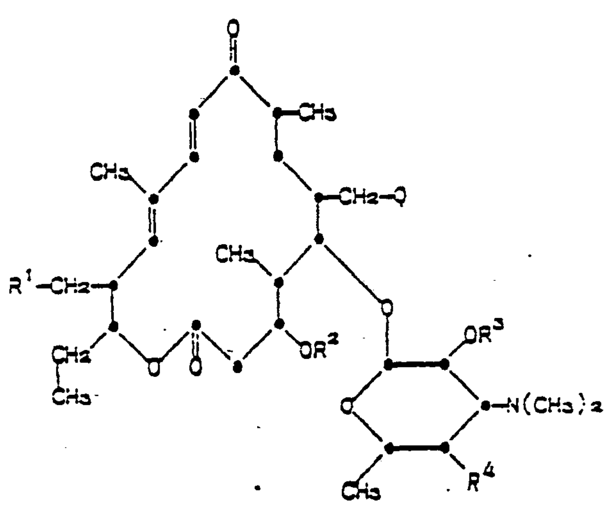

(II)

worin $R^1$, $R^2$, $R^3$ und $R^4$ wie in der Formel (I) definiert sind und Q für —CHO steht, durch Umsetzung mit einer Verbindung der Formel

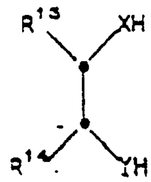 (XI) oder 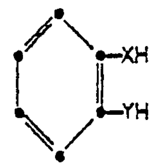 (XII)

worin $R^{13}$, $R^{14}$, X und Y wie in der Formel (I) definiert sind, in ein Makrolid der Formel (I) überführt wird, worin Z für folgende Gruppen steht

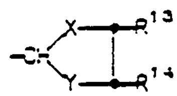 (VII)

oder

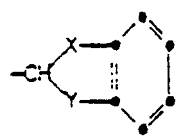 (VIII)

oder ein Makrolid der Formel (I), worin $R^4$ Mycarosyloxy bedeutet, in einer sauren Lösung bei einem pH-Wert von unter 4 hydrolysiert und so ein entsprechendes Makrolid der Formel (I) gebildet wird, worin $R^4$ Hydroxy ist,
oder ein Makrolid der Formel (I), worin $R^4$ ein Sulfonat ist, mit einer Quelle für Iodidionen umsetzt und so ein Makrolid der Formel (I) gebildet wird, worin $R^4$ Iod ist,
oder ein Makrolid der Formel (I), worin $R^4$ Iod ist, reduktiv deiodiert und so ein Makrolid der Formel (I) gebildet wird, worin $R^4$ Wasserstoff bedeutet, und
das nach irgend einer der vorhergehenden Stufen erhaltene Makrolid gegebenenfalls verestert oder in ein Salz überführt wird.

3. Makrolid der Formel (I) oder ein pharmazeutisch annehmbares Salz hiervon nach Anspruch 1 zur Verwendung als Antibiotikum bei der chemotherapeutischen Behandlung warmblütiger Tiere.

4. Futtervormischung, dadurch gekennzeichnet, daß sie ein Makrolid der Formel (I) oder ein pharmazeutisch annehmbares Salz hiervon nach Anspruch 1 als Wirkstoff enthält.

5. Veterinäre Formulierung, dadurch gekennzeichnet, daß sie ein Makrolid der Formel (I) oder ein pharmazeutisch annehmbares Salz hiervon nach Anspruch 1 als Wirkstoff in Verbindung mit ein oder mehr physiologisch annehmbaren Trägern oder Schlepplitteln hierfür enthält.

## Revendications

1. Macrolide de formule (I)

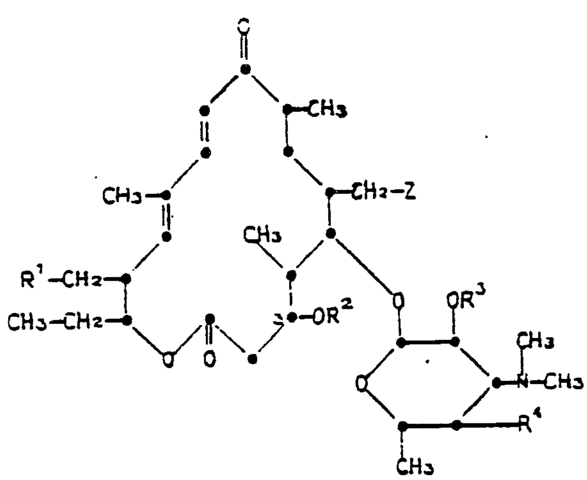 (I)

dans laquelle

$R^1$ représente :

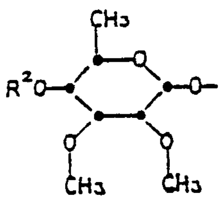 ; 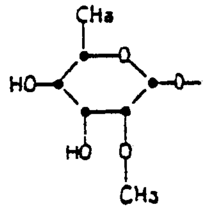 ; ou 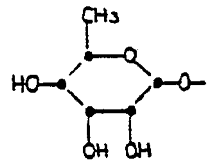

$R^2$ représente un atome d'hydrogène ou un groupe protecteur du groupe hydroxyle ;

$R^3$ représente un atome d'hydrogène, un groupe alcanoyle en $C_1$-$C_5$ éventuellement substitué, un groupe benzoyle éventuellement substitué, un groupe phénylacétyle éventuellement substitué ou un groupe phénylpropionyle éventuellement substitué ;

$R^4$ représente un atome d'hydrogène, un atome d'iode, un groupe hydroxyle, un groupe alcanoyl (en $C_1$-$C_5$)oxy éventuellement substitué, un groupe benzoyloxy éventuellement substitué, un groupe phénylacétoxy éventuellement substitué ou un groupe phénoxyacétoxy éventuellement substitué ou un groupe

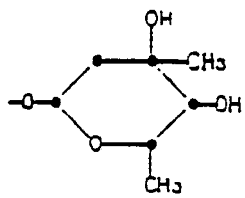

(mycarosyloxy)

Z représente un groupe

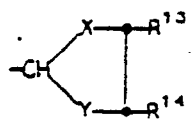

ou

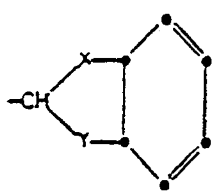

dans laquelle X et Y représentent chacun, indépendamment l'un de l'autre, O, S, NH, $NCH_3$, un groupe N-phényle, ou un groupe N-benzyle, tandis que $R^{13}$ et $R^{14}$ représentent chacun, independamment l'un de l'autre, un atome d'hydrogène, un groupe méthyle, un groupe phényle, un groupe méthoxycarbonyle, un groupe éthoxycarbonyle, ou un groupe phénoxycarbonyle, avec cette réserve que si $R^4$ représente un atome d'hydrogène ou un atome d'iode, alors $R^1$ représente

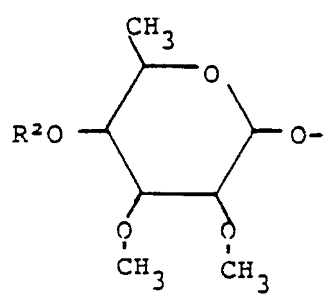

et avec cette réserve que $R^2$ représente un atome d'hydrogène, à moins que $R^4$ ne représente un atome d'hydrogène ou un atome d'iode.

2. Procédé de préparation d'un macrolide de formule (I) ou d'un de ses sels pharmaceutiquement acceptables, ce procédé consistant à faire réagir un macrolide de départ de formule (II) :

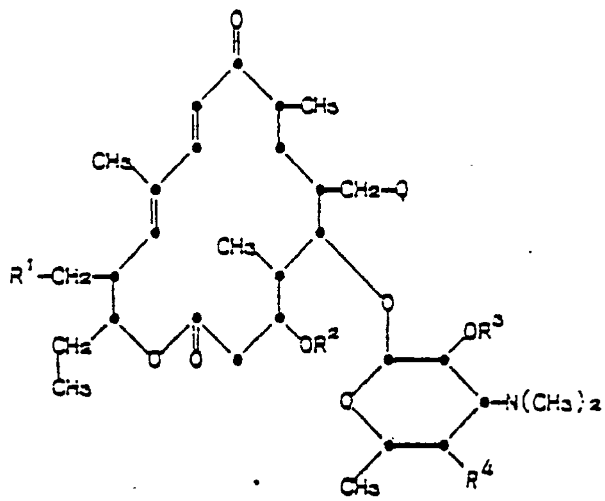

(II)

dans laquelle $R^1$, $R^2$, $R^3$, et $R^4$ ont les significations définies dans la formule (I), et où Q représente —CHO, avec un composé de formule

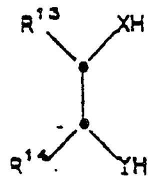

(XI) ou

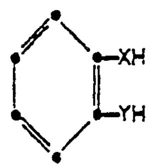

(XII)

où $R^{13}$, $R^{14}$, X et Y ont les significations définies dans la formule (I), pour obtenir un macrolide de formule (I), dans laquelle Z représente

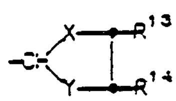

(VII)

ou

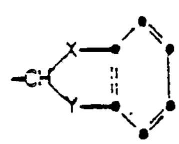

(VIII)

ou hydrolyser un macrolide de formule (I), dans laquelle $R^4$ représente un groupe mycarosyloxy, dans une solution acide à un pH inférieur à 4, pour obtenir un macrolide correspondant de formule (I), dans laquelle $R^4$ représente un groupe hydroxyle : ou faire réagir un macrolide de formule (I), dans laquelle $R^4$ représente un sulfonate, avec une source d'ions iodures, pour obtenir un macrolide de formule (I), dans laquelle $R^4$ représente un atome d'iode ; ou désioder par réduction un macrolide de formule (I), dans laquelle $R^4$ représente un atome d'iode, pour obtenir un macrolide de formule (I), dans laquelle $R^4$ représente un atome d'hydrogène ; et éventüellement estérifier ou salifier le macrolide obtenu via l'une quelconque des étapes précédentes.

3. Macrolide de formule (I), ou un de ses sels pharmaceutiquement acceptables, selon la revendication (I), pour être utilisé come antibiotique dans la chimiothérapie d'animaux à sang chaud.

4. Pré-mélange d'aliments comprenant, comme ingrédient actif, un macrolide de formule (I) ou un de ses sels pharmaceutiquement acceptables, selon la revendication 1.

5. Formulation vétérinaire comprenant, comme ingrédient actif, un macrolide de formule (I) ou un de ses sels pharmaceutiquement acceptables, selon la revendication 1, en association avec un ou plusieurs véhicules ou supports physiologiquement acceptables, pour cet ingrédient.